# NEW EUROPEAN PATENT SPECIFICATION

(11) **EP 0 384 652 B2**
(45) Date of publication and mention of the opposition decision: **02.11.2000**
(45) Mention of the grant of the patent: 19.01.1994
(21) Application number: 90301655.8
(22) Date of filing: 15.02.1990
(51) Int. Cl.: C07C 51/12, C07C 53/08

(54) **Process for preparing carboxylic acids**
Verfahren zur Herstellung von Carbonsäuren
Procédé de préparation d'acides carboxyliques

(30) Priority: 23.02.1989 GB 8904125
(43) Date of publication of application: 29.08.1990
(73) Proprietor: THE BRITISH PETROLEUM COMPANY P.L.C., London EC2M 7BA (GB)
(72) Inventor: Jones, Michael David, Sunbury-om-Thames, Middelsex TW16 7LN (GB)
(74) Representative: Barlow, Michael Thomas

(56) References cited:
- EP-A- 0 161 874
- EP-A- 0 265 140
- GB-A- 2 121 794
- US-A- 4 007 130
- US-A- 4 733 006

## Description

The present invention relates to a process for preparing a carboxylic acid having (n + 1) carbon atoms (e.g. acetic acid where (n = 1)) from an alcohol having n carbon atoms (e.g. methanol) by liquid phase carbonylation in the presence of a rhodium catalyst and an iodide promoter.

The preparation of acetic acid by the rhodium catalysed, iodide promoted carbonylatlon of methanol at elevated temperature and pressure is a well known process which is operated industrially. This particular reaction is an example of a genus of carbonylation reactions in which any alcohol having n carbon atoms can be transformed into a carboxylic acid having (n + 1) carbon atoms by reaction with carbon monoxide. Such reactions are described further in for example GB 1233121 and Applied Industrial Catalysis, Vol 1 275-296 (1983). Acetic acid is itself a well known valuable commodity chemical.

On an industrial scale, the carbonylation of methanol to acetic acid is carried out under steady state conditions by continuously feeding methanol, carbon monoxide, catalyst, iodide promoter and recycled material to a carbonylation reactor whilst at the same time continuously withdrawing an acetic acid containing product stream. Under typical steady state conditions the carbonylation reaction is carried out in the presence of a standing quantity of 14-15% water to ensure good reaction rates (see EP 55618 and Ind. Eng. Chem. Prod. Res. Dev. 16 281-285 (1977)). The other main components in the reactor at steady state are acetic acid, methyl iodide and methyl acetate produced by the rapid esterification of methanol to acetic acid under the operating conditions.

It is known from EP 55618 and EP 161874 that a problem with such a process is that the rhodium catalyst tends to precipitate in those parts of the plant where the carbon monoxide overpressure is relatively low or in situations where the level of standing water in the reactor is less than the norm. This latter phenomenon causes a decrease in reactor productivity, if the process is operated with lower levels of water.

EP 161874 discloses that this problem can be overcome by the use of a catalyst stabiliser such as a soluble metal iodide or quaternary iodide salt. Especially suitable salts are the alkali metal iodides (e.g. lithium iodide) since these are the most soluble in the reaction medium. For low standing quantities of water, e.g. 1-4% by weight, 10-20% by weight of the iodide salt has been found particularly beneficial. It has also been found that productivities at low water levels are improved further if the reactor is operated under conditions such that, at steady state, the reactor contains 2-5% by weight methyl acetate. This is higher than conventionally used in industrially operated plants.

Whilst the suggestion described above provides a way of obtaining higher acetic acid productivity from a reactor operating with low standing water levels it is obviously desirable to increase productivity further if possible. This is particularly true since, even in the presence of a catalyst stabiliser, the productivity is still dependent to a certain extent upon the level of water. Thus, in view of the above, the problem to be solved is to improve acetic acid productivity from a reactor operating at low water levels with a catalyst stabiliser.

One solution to this problem, described in EP 0250189, is to add at least 4 psig of hydrogen gas to the carbonylation reactor. Another solution which has now been discovered is to add effective amounts of a Group VIB metal costabiliser.

According to the present invention there is provided a continuous liquid-phase process for preparing a carboxylic acid having (n + 1) carbon atoms by reaction of carbon monoxide with an alcohol having n carbon atoms in the presence of a rhodium catalyst at elevated temperature and pressure which process comprises feeding the alcohol and/or an ester of the alcohol and the carboxylic acid together with carbon monoxide to a carbonylation reactor and removing the carboxylic acid from the carbonylation reactor characterised in that the carbonylation reactor contains during the course of the process a liquid reaction medium comprising:
(a) water at a concentration in the range 0.5 to 5 % by weight of the total weight of the reactor contents,
(b) a catalyst stabiliser selected from iodide salts which are soluble in the reaction medium at the temperature of the reaction,
(c) a Group VIB metal costabiliser,
(d) the iodide derivative of the alcohol,
(e) the ester of the carboxylic acid and the alcohol,
(f) a rhodium catalyst, and
(g) the carboxylic acid
   and in which process there is maintained a concentration in the reaction medium of the Group VIB costabiliser by adding to the reaction medium of an effective amount of a Group VIB metal costabiliser or by selectively removing all the corrosion metals with the exception of chromium, molybdenum or tungsten salts from the reaction medium.

The invention solves the problem defined above by employing a Group VIB metal costabiliser to enhance the rate of acetic acid production. Whilst not wishing to be bound by any theory, preliminary evidence from infrared spectroscopy suggests that the presence of the Group VIB metal costabiliser increases the concentration of catalytically active rhodium species Rh(CO)₂I₂⁻ in the carbonylation reactor relative to other less reactive or inactive forms.

The process which comprises the present invention can be applied to the conversion of any alcohol having n carbon atoms into any carboxylic acid having (n + 1) carbon atoms. The process is especially suitable, however, when an alcohol having from one to six carbon atoms is to be carbonylated. Preferably, the process is employed to convert either methanol to acetic acid or ethanol to propionic acid with the former of these being the most preferred. Whilst, for the rest of the description, the invention will be discussed in terms of the methanol to acetic acid process, it will be appreciated that all statements are applicable to other alcohol feedstocks with the appropriate obvious modifications.

As regards the rhodium catalyst, it is now well known that initially any rhodium source, including the metal itself, salts, complexes etc, can be introduced into the carbonylation reactor. This is because, under the reaction conditions, all such sources are converted into a common catalytically active species. It is believed that this species is the anion Rh(CO)₂I₂⁻.

Whilst the process can be operated at a carbon monoxide partial pressures as low as 1 atmosphere, a preferred range of partial pressures for industrial operation is from 2 to 100, most preferably 5 to 20 atmospheres. The process is preferably carried out at a temperature in the range 70 to 220°C, most preferably 140 to 190°C.

The process of the present invention is suitably operated by (a) continuously feeding methanol, methyl acetate or mixtures thereof to a carbonylation reactor together with recycle streams, carbon monoxide and optionally hydrogen and (b) continuously withdrawing an acetic acid containing product stream. During such continuous operation the reactor is maintained at a steady state composition in which the primary components in the liquid phase are methyl acetate, methyl iodide, hydrogen iodide, acetic acid, water, rhodium, catalyst stabiliser and Group VIB metal costabiliser. Secondary components, e.g. side products etc may also be present in small amounts. Preferably the steady state composition of the liquid phase is such that the primary components, excluding acetic acid, which together with impurities and the secondary components constitute the balance of the liquid phase, fall within the following broad and preferred ranges:-

| | Broad Wt% | Preferred Wt% |
|---|---|---|
| Water | 0.1 - 15 | 0.5 - 5 |
| Methyl Acetate | 0.1 - 6 | 1 - 6 |
| Methyl Iodide | 5 - 20 | 10 - 16 |
| Rhodium (ppm) | 100 - 1000 | 250 - 750 |
| Catalyst Stabiliser (as LiI) | 2 - 20 | 10 - 20 |
| Group VIB Costabiliser (as Mo(CO)₆) (ppm) | 0 - 10,000 | 1 - 5000 |

The catalyst stabiliser, as is indicated in EP 161874, can be any metal iodide or quaternary ammonium iodide salt. Preferred examples of the metal iodide salts are the alkali and alkaline earth metals including lithium iodide, sodium iodide, potassium iodide, caesium iodide, magnesium iodide and calcium iodide. Such iodide salts are preferred because they have reasonable solubility in the reaction medium. It is believed that in the case of the alkali and alkaline earth metal iodides the nature of the cation is relatively unimportant and that it is the presence of solubilised iodide ion in the reaction medium that causes the improvement. That being the case, the most preferred metal iodide is lithium iodide which has the greatest solubility in the reaction medium.

The metal iodide salt can be introduced directly into the carbonylation reactor. Alternatively the iodide salt can be generated in situ since under the operating conditions of the carbonylation reactor a wide range of metal salts will react with methyl iodide to general the metal iodide. Preferred metal salts which can be used in this way are the C₁ to C₆ carboxylate salts eg the acetate or propionate salts.

Soluble quaternary ammonium or phosphonium iodides can be used as alternatives to the metal iodides. These iodides can also be either added directly or generated in situ. Typical quaternary ammonium iodides are those where the cation has been obtained by quaternisation of a tertiary amine, pyridine, pyrolidine, imidazole or other heterocyclic nitrogen containing compound.

The Group VIB metal costabilisers can be any of the metals designated as being in Group VIB of the Periodic Table as defined in Cotton and Wilkinson "Advanced Inorganic Chemistry", 3rd Edition, ie chromium, molybdenum and tungsten most preferably molybdenum and tungsten. Whilst these metals can be added to the reaction medium in any form, it is preferred to use a low valent salt, such as molybdenum, chromium or tungsten acetate or iodide, or a carbonyl complex such as Mo(CO)₆, W(CO)₆, Cr(CO₆) and the like.

The behaviour of the Group VIB metals in further increasing acetic acid productivity is surprising and not a phenomenon apparently shared by all other transition metals. A case in point is iron, the effect of which is to actually reduce reactor productivity. This discovery is particularly important because on an industrial plant corrosion leads to a build up of iron, molybdenum, tungsten, chromium and nickel salts in the reactor. It would appear that this build up of such metal salts has previously been viewed as detrimental, presumably because of the presence of the iron. Thus, previously, methods have been developed to remove all corrosion metals from the reaction medium (e.g. US 4628041 and EP 265146). It is now however apparent that there is no need to remove chromium, molybdenum and tungsten from the reaction medium.

Hence, in an embodiment of the present invention, there is provided a process for treating process streams, arising in the manufacture of acetic acid from methanol, which process streams contain rhodium and typical corrosion metal salts characterised in that the process comprises selectively removing all the corrosion metals from the process stream with the exception of chromium, molybdenum or tungsten salts.

Selective removal of for example iron from a process stream containing rhodium, iron, chromium, molybdenum and tungsten can be achieved by the use of selective ion exchange resins, electrodialysis, selective precipitation and the like. The process defined by the embodiment is of course also applicable to process streams which arise during the carbonylation of any n carbon atom alcohol to (n + 1) carbon atom carboxylic acid.

The invention is now illustrated by the following Examples.

### Example 1 - Use of Molybdenum Carbonyl as Costabiliser

A 150 ml Hastelloy autoclave was charged with AcOH (32.2g), MeOAc (17.7g), H₂O (2.95g), MeI (28.1g), LiOAc.2H₂O (8.609g), and a solution containing Mo(CO)₆ (0.82g), aq.HI (57% solution, 1.45g) and AcOH (4.0g). The autoclave was sealed, purged with N₂ (3 x 40barg), pressurised with N₂ (3 barg) and heated to 185°C and allowed to stabilise for 25 minutes. To the autoclave was injected 35 [RhCl(CO)₂]₂ (0.06g) in AcOH (5.84g) under a pressure of CO. The CO was continually fed via a pressure regulating valve such that the autoclave pressure remained constant at about 36 barg. CO was fed from a ballast vessel and the pressure was measured using a transducer reading to 0.1 bar. The gas uptake rate at a position corresponding to a solution composition of MeOAc 5% w/w and H₂O (2% w/w) was measured as 7.6 mol/1h).

### Comparative Experiment A - No Group VI B Metal Present

A 150 ml Hastelloy autoclave was charged with AcOH (38.2g), MeOAc (17.76g), H₂O (3.56g), MeI (27.9g) and LiOAc.2H₂O (8.60g). The autoclave was sealed, purged and heated to 185°C as described in Example 1. The catalyst solution [RhCl(CO)₂]₂ (0.06g) in AcOH (5.84g) was injected into the autoclave using an overpressure of CO which was again fed to the autoclave on demand via a back pressure regulator from a reservoir vessel. The gas uptake rate at a position corresponding to a solution composition of MeOAc (5% w/w) and H₂O (2% w/w) was measured as 5.9 mol/1h.

### Comparative Experiment B - Effect of Iron

A 150 ml Hastelloy autoclave was charged with AcOH (32.2g), MeOAc (17.7g), H₂O (2.2g), MeI (27.9g), LiOAc.2H₂O (8.6g) and a solution containing basic iron acetate (1.3g), aqueous HI (57% solution 3.2g) and AcOH (3.0g). The autoclave was sealed, purged and heated to 185°C as described in Example 1. The catalyst solution [RhCl(CO)₂]₂ (0.06g) in AcOH (5.85g) was injected into the autoclave using an overpressure of CO. The gas uptake rate at a position corresponding to a solution composition of MeOAc (5% w/w) and H₂O (2% w/w) was measured as 1.9 mol/1h. This Example shows that not all corrosion metals have a beneficial effect on reaction rate.

### Example 2 - Use of Chromium Acetate as Costabiliser

A 150 ml Hastelloy autoclave was charged with AcOH (32.2g), MeOAc (17.66g), H₂O (3.1g), MeI (27.9g), LiOAc.2H₂O (8.6g) and a solution of chromium acetate (1.51g), aqueous HI (57% solution, 1.1g) and AcOH (4.0g). The autoclave was sealed, purged and heated to 185°C as described in Example 1. The catalyst solution [RhCl(CO)₂]₂ (0.06g) in AcOH (5.84g) was injected into the autoclave using an overpressure of CO. The gas uptake rate at the position corresponding to a solution composition of MeOAc (5% w/w) and H₂O (2% w/w) was measured as 6.5 mol/1h.

### Example 3 - Use of Tungsten Carbonyl as Costabiliser

The autoclave was charged with AcOH (32.2g), MeOAc (17.7g), H₂O (2.85g), MeI (27.9g), LiOAc.2H₂O (8.6g) and a solution of [W(CO)₆] (1.1g), aqueous HI (57% solution, 1.4g) and AcOH (4.0g). The autoclave was sealed, purged and heated to 185°C as described in Example 1. The catalyst solution [RhCl(CO₂]₂ (0.061g), in AcOH (5.84g) was injected into the autoclave using an overpressure of CO. The gas uptake rate at the position corresponding to a solution composition of MeOAc (5% w/w) and H₂O (2% w/w) was measured as 9.2 mol/1h.

### Example 4 - Use of Chromium Carbonyl as Costabiliser

The autoclave was charged with AcOH (32.2g), MeOAc (17.7g), H₂O (2.85g), MeI (27.9g), LiOAc.2H₂O (8.6g) and a solution of [Cr(CO)₆] (0.55g), aqueous HI (57% solution, 1.1g) and AcOH (4.0g). The autoclave was sealed, purged and heated to 185°C as described in Example 1. The catalyst solution [RhCl(CO₂]₂ (0.061g), in AcOH (5.84g) was injected into the autoclave using an overpressure of CO. The gas uptake rate at the position corresponding to a solution composition of MeOAc (5% w/w) and H₂O (2% w/w) was measured as 7.1 mol/1h.

### Example 5 - Use of Molybdenum Carbonyl as Costabiliser

A solution of [Mo(CO)₆] in AcOH (31.5g) was heated for 24 hours at 80°C in a sealed Schlenck tube. The resultant solution was cooled and transferred to an autoclave containing AcOH (4.8g), MeOAc (17.6g), H₂O (2.9g), MeI (27.9g), LiOAc.2H₂O (8.61g). The autoclave was sealed, purged and heated to 185°C as described in Example 1. The catalyst solution [RhCl(CO₂]₂ (0.061g), in AcOH (5.84g) was injected into the autoclave using an overpressure of CO. The gas uptake rate at the position corresponding to a solution composition of MeOAc (5% w/w) and H₂O (2% w/w) was measured as 8.1 mol/1h.

### Example 6 - Use of Molybdenum Carbonyl as Costabiliser

A 150 ml Hastelloy autoclave was charged with AcOH (32.2g), MeOAc (17.6g), H₂O (3.5g), MeI (28.5g), LiOAc.2H₂O (8.6g) and [Mo(CO)₆] (0.82g). The autoclave was sealed, purged and heated to 185°C as described in Example 1. The catalyst solution [RhCl(CO)₂]₂ (0.06g) in AcOH (5.84g) was injected into the autoclave using an overpressure of CO. The gas uptake rate at the position corresponding to a solution composition of MeOAc (5% w/w) and H₂O (2% w/w) was measured as 8.5 mol/1h.

### Example 7 - Use of Molybdenum Carbonyl as Costabiliser

A 150 ml Hastelloy autoclave was charged with AcOH (36.2g), MeOAc (17.65g), H₂O (3.55g), MeI (29.1g), LiOAc.2H₂O (8.6g) and [Mo(CO)₆] (1.64g). The autoclave was sealed, purged and heated to 185°C as described in Example 1. The catalyst solution [RhCl(CO)₂]₂ (0.06g) in AcOH (5.84g) was injected into the autoclave using an overpressure of CO. The gas uptake rate at the position corresponding to a solution composition of MeOAc (5% w/w) and H₂O (2% w/w) was measured as 7.9 mol/1h.

### Example 8 - Use of Molybdenum Carbonyl as Costabiliser

The autoclave was charged with AcOH (36.2g), MeOAc (17.65g), H₂O (3.5g), MeI (27.88g), LiOAc.2H₂O (8.6g) and [Mo(CO)₆] (0.41g). The autoclave was sealed, purged and heated to 185°C as described in Example 1. The catalyst solution [RhCl(CO₂]₂ (0.06g), in AcOH (5.84g) was injected into the autoclave using an overpressure of CO. The gas uptake rate at the position corresponding to a solution composition of MeOAc (5% w/w) and H₂O (2% w/w) was measured as 7.1 mol/1h.

### Example 9 - Use of Molybdenum Carbonyl as Costabiliser

The autoclave was charged with AcOH (36.2g), MeOAc (17.68g), H₂O (3.53g), MeI (28.Og), LiOAc.2H₂O (8.6g) and a [Mo(CO)₆] (0.14g). The autoclave was sealed, purged and heated to 185°C as described in Example 1. The catalyst solution [RhCl(CO₂]₂ (0.06g), in AcOH (5.84g) was injected into the autoclave using an overpressure of CO. The gas uptake rate at the position corresponding to a solution composition of MeOAc (5% w/w) and H₂O (2% w/w) was measured as 6.6 mol/1h.

### Example 10 - Use of Molybdenum Acetate as Costabiliser

The autoclave was charged with AcOH (32.2g), MeOAc (17.64g), H₂O (2.95g), MeI (28.0g), LiOAc.2H₂O (8.606g), and a solution of molybdenum acetate (0.67g), a.q. HI (57% solution 1.41g) and AcOH (4.0g). The autoclave was sealed, purged with N₂ and heated to 185°C as in Example 1. The catalyst solution [RhCl(CO)₂]₂ (0.06g) in AcOH (5.84g) was injected into the autoclave using an overpressure of CO. The gas uptake rate at the position corresponding to a solution composition of MeOAc (5% w/w) and H₂O (2% w/w) was measured as 7.6 mol/1h.

### Comparative Tests - Use of Vanadium Acetoacetonate

The autoclave was charged with AcOH (36.2g), MeOAc (17.66g), H₂O (3.55g), MeI (28.0g), LiOAc.2H₂O (8.60g), and V(acac)₂ (1.1g). The autoclave was sealed, heated to 185°C as in Example 1. The catalyst solution [RHCl(CO)₂]₂ (0.061g) in AcOH (5.84g) was injected into the autoclave using an overpressure of CO. The gas uptake rate measured at a solution composition of MeOAc (5% w/w) and H₂O (2% w/w) was measured as 5.9 mol/1h.

## Claims

1. A continuous liquid-phase process for preparing a acid having (n+1) carbon atoms by reaction of carbon monoxide with an alcohol having n carbon atoms in the presence of a rhodium catalyst at elevated temperature and pressure which process comprises feeding the alcohol and/or an ester of the alcohol and the carboxylic acid together with carbon monoxide to a carbonylation reactor and removing the carboxylic acid from the carbonylation reactor; characterised in that the carbonylation reactor contains during the course of the process a liquid reaction medium comprising :
(a) water at a concentration in the range 0.5 to 5 % by weight of the total weight of the reactor contents.
(b) a catalyst stabiliser selected from iodide salts which are soluble in the reaction medium at the temperature of the reaction,
(c) a Group VIB metal costabiliser,
(d) the iodide derivative of the alcohol,
(e) the ester of the carboxylic acid and the alcohol,
(f) a rhodium catalyst, and
(g) the carboxylic acid
and in which process there is maintained a concentration in the reaction medium of the Group VIB costabiliser by adding to the reaction medium of an effective amount of a Group VIB metal costabiliser or by selectively removing all the corrosion metals with the exception of chromium, molybdenum or tungsten salts from the reaction medium.

2. A liquid-phase process as claimed in claim 1 characterised in that the carboxylic acid is acetic acid and the alcohol is methanol.

3. A liquid-phase process as claimed in claim 2 characterised in that the level of methyl acetate in the reactor is 1 to 6% of the total weight of the reactor contents.

4. A liquid-phase process as claimed in claim 3 characterised in that the catalyst stabiliser is an alkali metal iodide.

5. A liquid-phase process as claimed in claim 4 characterised in that the Group VIB metal costabiliser is either a molybdenum or tungsten costabiliser.

6. A liquid-phase process as claimed in claim 5 characterised in that the Group VIB metal costabiliser is a low valent molybdenum or tungsten salt or a molybdenum or tungsten carbonyl.

## Patentansprüche

1. Kontinuierliches Flüssigphasen-Verfahren zur Herstellung einer Carbonsäure mit (n + 1) Kohlenstoffatomen durch Reaktion von Kohlenmonoxid mit einem Alkohol mit n Kohlenstoffatomen in Gegenwart eines Rhodiumkatalysators bei erhöhter Temperatur und Druck, welches das Einspeisen des Alkohols und/oder eines Esters des Alkohols und der Carbonsäure, zusammen mit Kohlenmonoxid, in einen Carbonylierungsreaktor, und das Entfernen der Carbonsäure aus dem Carbonylierungsreaktor, umfaßt, dadurch gekennzeichnet, daß der Carbonylierungsreaktor während des Ablaufs des Verfahrens ein flüssiges Reaktionsmedium enthält, umfassend:
(a) Wasser in einer Konzentration im Bereich von 0,5 bis 5 Gew.-% des Gesamtgewichts der Reaktorinhalte,
(b) einen Katalysator-Stabilisator, ausgewählt aus Iodidsalzen, die in dem Reaktionsmedium bei der Reaktionstemperatur löslich sind,
(c) einen Metall-Costabilisator der Gruppe VIB,
(d) das Iodid-Derivat des Alkohols,
(e) den Ester der Carbonsäure und des Alkohols,
(f) einen Rhodiumkatalysator und
(g) die Carbonsäure
und wobei in dem Verfahren in dem Reaktionsmedium eine Konzentration des Costabilisators der Gruppe VIB beibehalten wird, durch Zugabe einer wirksamen Menge eines Metall-Costabilisators der Gruppe VIB zu dem Reaktionsmedium oder durch selektive Entfernung aller Korrosionsmetalle mit Ausnahme von Chrom-, Molybdän- oder Wolframsalzen aus dem Reaktionsmedium.

2. Flüssigphasen-Verfahren nach Anspruch 1, dadurch gekennzeichnet, daß die Carbonsäure Essigsäure und der Alkohol Methanol ist.

3. Flüssigphasen-Verfahren nach Anspruch 2, dadurch gekennzeichnet, daß die Menge des Methylacetats in dem Reaktor 1 bis 6% des Gesamtgewichts der Reaktorinhalte beträgt.

4. Flüssigphasen-Verfahren nach Anspruch 3, dadurch gekennzeichnet, daß der Katalysator-Stabilisator ein Alkalimetalliodid ist.

5. Flüssigphasen-Verfahren nach Anspruch 4, dadurch gekennzeichnet, daß der Metall-Costabilisator der Gruppe VIB entweder ein Molybdän- oder ein Wolfram-Costabilisator ist.

6. Flüssigphasen-Verfahren nach Anspruch 5, dadurch gekennzeichnet, daß der Metall-CostabilisatOr der Gruppe VIB ein niedrigwertiges Molybdän- oder Wolframsalz oder ein Molybdän- oder Wolframcarbonyl ist.

## Revendications

1. Procédé continu en phase liquide pour la préparation d'un acide carboxylique ayant (n + 1) atomes de carbone par réaction de monoxyde de carbone avec un alcool ayant n atomes de carbone en présence d'un catalyseur au rhodium à température et sous pression élevées, procédé qui comprend l'introduction de l'alcool et/ou d'un ester de l'alcool et de l'acide carboxylique conjointement avec du monoxyde de carbone dans un réacteur de carbonylation, et l'évacuation de l'acide carboxylique du réacteur de carbonylation, caractérisé en ce que le réacteur de carbonylation contient, au cours de la mise en oeuvre du procédé, un milieu réactionnel liquide comprenant :
(a) de l'eau à une concentration comprise dans l'intervalle de 0,5 à 5 % en poids du poids total du contenu du réacteur,
(b) un stabilisant de catalyseur choisi entre des iodures qui sont solubles dans le milieu réactionnel à la température de la réaction,
(c) un costabilisant contenant un métal du Groupe VIB,
(d) l'iodure dérivé de l'alcool,
(e) l'ester de l'acide carboxylique et de l'alcool,
(f) un catalyseur au rhodium, et
(g) l'acide carboxylique,
procédé dans lequel est maintenue une concentration dans le milieu réactionnel du costabilisant du Groupe VIB en ajoutant au milieu réactionnel une quantité efficace d'un costabilisant contenant un métal du Groupe VIB ou en éliminant sélectivement tous les métaux sensibles à la corrosion, à l'exception des sels de chrome, de molybdène ou de tungstène du milieu réactionnel.

2. Procédé en phase liquide suivant la revendication 1, caractérisé en ce que l'acide carboxylique est l'acide acétique et l'alcool est le méthanol.

3. Procédé en phase liquide suivant la revendication 2, caractérisé en ce que la quantité d'acétate de méthyle dans le réacteur est comprise dans l'intervalle de 1 à 6 % du poids total du contenu du réacteur.

4. Procédé en phase liquide suivant la revendication 3, caractérisé en ce que le stabilisant de catalyseur est un iodure de métal alcalin.

5. Procédé en phase liquide suivant la revendication 4, caractérisé en ce que le costabilisant contenant un métal du Groupe VIB est un costabilisant consistant en molybdène ou tungstène.

6. Procédé en phase liquide suivant la revendication 5, caractérisé en ce que le costabilisant contenant un métal du Groupe VIB est un sel de molybdène ou de tungstène de valence faible ou un dérivé carbonylé de molybdène ou de tungstène.
